# EUROPÄISCHE PATENTANMELDUNG

(11) **EP 0 995 427 A2**
(43) Veröffentlichungstag der Anmeldung: **26.04.2000**
(21) Anmeldenummer: 99115886.6
(22) Anmeldetag: 12.08.1999
(51) Int. Cl.: A61K 7/48

(54) **Grüntee-Extrakt enthantende O/W-Emulsionen**

(30) Priorität: 07.09.1998 DE 19840730
(71) Anmelder: Dragoco Gerberding & Co Aktiengesellschaft, D-37603 Holzminden (DE)
(72) Erfinder: Lange, Sabine, 37603 Holzminden (DE); Pickenhagen, Wilhelm, Dr., 37671 Höxter (DE)
(74) Vertreter: Eikenberg & Stilkenböhmer

(57) **Zusammenfassung**

Beschrieben wird eine O/W-Emulsion mit einen Gehalt an Grüntee-Extrakt, die insbesondere zur kosmetischen oder pharmazeutischen Verwendung geeignet ist. Der pH-Wert der Emulsion liegt dabei zwischen 2 und 6, und sie umfaßt einen mehrzähnigen Komplexliganden, wobei der als Maß für die Helligkeit der Emulsion anzusehende L-Wert über 81 liegt. Es handelt sich also um eine sehr helle O/W-Emulsion.

Vorzugsweise handelt es sich bei dem Komplexliganden um Ethylendiamintetraessigsäure oder Ethylendiamintetraacetat.

Die beschriebenen hellen O/W-Emulsionen sind über lange Zeit farbstabil.

## Beschreibung

Die Erfindung betrifft helle O/W-Emulsionen, die einen Grüntee-Extrakt enthalten und zwar insbesondere kosmetische oder pharmazeutische O/W-Emulsionssysteme.

Grüner Tee (camellia sinensis) erfreut sich beim Verbraucher großer Beliebtheit, da der Genuß eines Grüntee-Extraktes als Getränk mit zahlreichen positiven Wirkungen auf den Körper verbunden ist; hervorzuheben sind dabei sowohl belebende als auch antioxidierende Wirkungen.

Als insoweit wesentliche Inhalts- und Wirkstoffe des Grüntee-Extrakts werden neben Coffein die Polyphenole (-)-Epigallocatechin, (+)-Catechin, (-)-Epicatechin, (-)-Epigallocetechingallat, (-)-Gallocatechingallat und (-)-Epicatechingallat angesehen.

Die positiven Wirkungen des Grünen Tees sind nicht auf die Anwendung als Nahrungsmittel beschränkt. Insbesondere die antioxidierenden Eigenschaften des Grünen Tees sind auch für kosmetische oder pharmazeutische Formulierungen, insbesondere für die kosmetische Hautanwendung, von besonderer technischer und kommerzieller Bedeutung. So ist beispielsweise bekannt, daß reaktive Sauerstoffspezies, wie z. B. das Hydroxylradikal, aber auch Singulettsauerstoff, eine wichtige Rolle bei Entzündungsprozessen und bei der Hautalterung (insbesondere der Faltenbildung) spielen. Durch Einsatz von Polyphenolen aber, also zum Beispiel durch den Einsatz der oben genannten Inhaltsstoffe des Grünen Tees, kann ein schnelles Quenchen derartiger reaktiver Sauerstoffspezies erreicht werden; die Polyphenole wirken dabei als Antioxidans. Durch geeignete Applikation von Polyphenolen auf Basis von Grünem Tee kann man somit negativen Hautveränderungen wirkungsvoll begegnen und einen gesunden Hautzustand lange erhalten.

Um den Grünen Tee für diese Zwecke nutzbar zu machen, müssen dessen Inhaltsstoffe mit einem geeigneten Lösungsmittel extrahiert werden. Eine Extraktion kann dabei beispielsweise mit heißem Wasser oder kurzkettigen Alkoholen geschehen. Ein wässriger Grüntee-Extrakt kann sehr wirtschaftlich durch Sprühtrocknung zu einem bräunlichen Pulver aufkonzentriert werden. Dieses Extrakt-Pulver ist ohne feststellbare Veränderungen lange lagerfähig und liefert beim Versetzen mit Wasser eine Extrakt-Lösung zurück. In seiner Zusammensetzung ähnelt oder entspricht ein Wasserextrakt des Grünen Tees natürlich einem Aufguß, wie ihn ein Konsument beim üblichen Zubereiten eines Getränks auf Basis von Grünem Tee erhält.

Um die Vorteile eines Grüntee-Extraktes auch für die Hautpflege nutzbar zu machen, müssen geeignete Formulierungen zur Anwendung auf der Haut zur Verfügung gestellt werden.

Besonders vorteilhaft erscheint a priori eine Einarbeitung von Grüntee-Extrakten in O/W-Emulsionssysteme, da diese eine generell hohe Akzeptanz bei Verbraucher besitzen. Eine Einarbeitung von Grüntee-Extraktkonzentraten in O/W-Emulsionen ist auch problemlos möglich.

Probleme ergeben sich jedoch bei der Lagerung solcher einen Grüntee-Extrakt enthaltender O/W-Emulsionen, und zwar umso stärker, je heller die Emulsionen sind. Es kommt nämlich bei der Lagerung allmählich zu einer Dunkelfärbung oder Farbvertiefung der Formulierung, die vom Verbraucher als Verderb interpretiert wird. Die Dunkelfärbung von hellen O/W-Formulierungen, die einen Grüntee-Extrakt umfassen, steht demzufolge ihrer erfolgreichen Vermarktung im Wege. Die Verbraucher erwarten, daß die helle Farbe der Formulierung auch über den gesamten Verwendungszeitraum hinweg unverändert bestehen bleibt. Bei O/W-Emulsionen, die aufgrund von farbigen Zusätzen eine dunkle Originalfarbe besitzen, treten diese Probleme nicht in Erscheinung, da dort eine sich bei der Lagerung verstärkende Dunkelfärbung vom Verbraucher praktisch nicht bemerkt wird. Da die Verbraucher jedoch im Allgemeinen eine helle Farbe der Formulierung bevorzugen, besteht ein Bedarf an Maßnahmen, welche die allmähliche Dunkelfärbung von hellen O/W-Emulsionen verhindern oder zumindest vermindern.

Es war deshalb die Aufgabe der vorliegenden Erfindung, eine einen Grüntee-Extrakt umfassende O/W-Emulsion zur Verfügung zu stellen, bei der eine Verfärbung nicht oder zumindest nur verzögert stattfindet. Mit anderen Worten sollte die optische Lagerstabilität derartiger Emulsionen verbessert werden.

Erfindungsgemäß wird diese Aufgabe gelöst durch eine O/W-Emulsion mit einem Gehalt an Grüntee-Extrakt, welche sich dadurch kennzeichnet,
- daß der pH-Wert der Emulsion zwischen 2 und 6 liegt,
- daß die Emulsion einen mehrzähnigen Komplexliganden umfaßt und
- daß die Emulsion einen L-Wert über 81 aufweist.

Überraschenderweise hat sich gezeigt, daß bei einer erfindungsgemäß beschaffenen Emulsion die Verfärbungsgeschwindigkeit deutlich reduziert ist. Eine Formulierung mit einem erfindungsgemäßen pH-Wert und mit einen Gehalt an einem mehrzähnigen Komplexliganden erscheint dem Verbraucher daher noch zu einem Zeitpunkt unverfärbt und hell, an dem eine ansonsten identische, zur gleichen Zeit hergestellte Formulierung mit einem höheren pH-Wert bzw. ohne einem mehrzähnigen Komplexliganden bereits eine störende Brauntönung aufweist.

Der L-Wert ist eine mit Farbmeßgeräten nach CIELAB, Din 6174 aus dem von einer beleuchteten Probe reflektierten Licht gewonnener Meßzahl, welche die Helligkeit der Probe angibt. Je größer diese Zahl ist, desto heller ist die Probe. Ein L-Wert von 81 liegt bereits verhältnismäßig nahe am Idealwert für Reinweiß (L=100) und bedeutet damit eine helle weiße Emulsion. Der L-Wert einer erfindungsgemäß beschaffenen Emulsion sinkt auch bei Lagerungszeiten von mehreren Monaten nicht unter 81 ab.

Aus der Literatur bekannt ist, daß unter sauren Bedingungen Proanthocyanidine, wie sie auch im Grünen Tee vorkommen, zu farbigen Anthocyanidinen umgewandelt werden. Unter sauren Bedingungen ist daher nach längeren Standzeiten eine Dunkelfärbung von wässrigen Phasen zu erwarten, die einen Grüntee-Extrakt umfassen. Nachteiligerweise fördert ein saures Milieu überdies Polykondensations Reaktionen monomerer Polyphenole zu Proanthocyanidinen, die sich dann auf die beschriebene Weise in die farbigen Anthocyanidine umwandeln. Überraschenderweise ist eine erfindungsgemäße Emulsion mit einem pH zwischen 2 und 6 jedoch farbstabiler als eine ansonsten identische Formulierung mit einem höheren pH-Wert. Es ist anzunehmen, daß sich hierbei der mehrzähnige Komplexligand stabilisierend auswirkt.

Hervorzuheben ist, daß es sich bei dem Gegenstand der Erfindung um eine O/W-Emulsion handelt, und nicht um eine beliebige sonstige Formulierung. Vermutlich ist eine positive Wechselwirkung der Polyphenolkompenten des Grüntee-Extrakts mit dem Gelnetzwerk der Emulsion für die Farbstabilisierung mitverantwortlich.

Wenngleich im gesamten pH-Intervall zwischen pH 2 und pH 6 eine sehr hohe optische Lagerstabilität der erfindungsgemäßen Emulsionen gewährleistet ist, sind doch bestimmte pH-Intervalle bevorzugt. Eine besonders gute Farbstabilität wird bei pH-Werten zwischen 2 und 4 erreicht. In diesem pH-Bereich ist jedoch für die Haut eine Reizwirkung durch die Säure nicht auszuschließen. Ein pH-Wert zwischen 2 und 4 wird daher regelmäßig nur dann eingestellt werden, wenn die Säurewirkung tolerabel oder sogar erwünscht ist, z. B. um eine Peeling-Aktivität zu erhalten. Hautpflege- und sonstige Formulierungen, die keine Reizwirkung auf die Haut besitzen sollen, werden dagegen vorzugsweise so eingestellt, daß sowohl eine ausreichende Farbstabilität als auch eine gute Hautverträglichkeit erreicht werden. Als besonders günstig haben sich hier Emulsionen mit einem pH zwischen 4,5 und 5,5 erwiesen.

Als mehrzähnige Komplexliganden werden bevorzugt Ethylendiamintetraessigsäure (EDTA) und Ethylendiamintetraacetat eingesetzt, mit denen, wie weiter unten noch näher erläutert werden wird, besonders gute Resultate erzielt worden, insbesondere wenn der pH-Wert zwischen 4,5 und 5,5 liegt.

Nachfolgend wird die Erfindung anhand von Beispielen näher erläutert.

### Beispiel 1: Farbstabile O/W-Creme (pH 5)

### Rezeptur mit Grünem Tee und EDTA bei pH 5

Die Creme wird aus vier Phasen gebildet, deren Zusammensetzung nachstehend angegeben ist:

| Zusammensetzung Phase A | |
|---|---|
| **Rohstoffe [INCI Bezeichnungen] (Lieferant)** | **Anteil [%]** |
| Glyceryl Stearate, PEG-100 Stearate (Dracorin 100 S.E. P, DRAGOCO) | 8,0 |
| Glyceryl Stearate (Dracorin GMS, DRAGOCO) | 2,0 |
| Paraffinum Liquidum | 4,0 |
| Certyl Alcohol | 2,0 |
| Isopropyl Myristate | 4,0 |
| Cetearyl Octanoate, Isopropyl Myristate (PCL Liquid, DRAGOCO) | 4,0 |
| Dimethicone | 0,5 |

| Zusammensetzung Phase B | |
|---|---|
| Aqua | 68,2 |
| Phenoxyethanol, Methylparaben, Ethylparaben, Butylparaben, Propylparaben, Isobutylparaben (Dragocid Liquid, DRAGOCO) | 0,8 |
| Trisodium Ethylendiamin Tetraacetate | 0,1 |
| Propylene Glycol | 5,0 |

| Zusammensetzung Phase C | |
|---|---|
| Aqua | 1,0 |
| Camellia Sinensis (Grüner Tee Extra, DRAGOCO) | 0,1 |

| Zusammensetzung Phase D | |
|---|---|
| Fragrance | 0,3 |

Zur Herstellung der Emulsion werden die Phasen A und B getrennt auf ca. 80 °C erhitzt. Die Phase C wird in der Phase B gelöst. Unter starkem Dispergieren wird die Phase A zu den vereinigten Phasen B+C gegeben. Unter ständigem Rühren mit einem Blattrührer wird die Emulsion abgekühlt. Bei 40 °C wird Phase D zugesetzt. Der pH-Wert der fast weißen Emulsion beträgt 5,0.

### Beispiel 2: Nicht-farbstabile O/W-Creme (pH 6,6)

### Rezeptur mit Grünem Tee und EDTA bei pH 6,6.

Die Creme wird unter Verwendung von vier Phasen A-D gebildet, deren Zusammensetzung der aus Beispiel 1 entspricht.

Zur Herstellung der Emulsion werden die Phasen A und B getrennt auf ca. 80 °C erhitzt. Die Phase C wird in der Phase B gelöst. Unter starkem Dispergieren wird die Phase A zu den vereingten Phasen B+C gegeben. Unter ständigem Rühren mit einem Blattrührer wird die Emulsion abgeühlt. Die Phase D wird bei 40 °C zugesetzt. Der pH-Wert der fast weißen Emulsion wird mit Natronlauge auf pH = 6,6 eingestellt.

### Beispiel 3: O/W-Creme ohne EDTA (pH 5)

### Rezeptur mit Grünem Tee bei pH 5

Die Creme wird unter Verwendung von vier Phasen A, B₁, C und D gebildet, wobei die Zusammensetzung der Phasen A, C und D der aus den Beispielen 1 und 2 entspricht.

| Zusammensetzung Phase B₁ | |
|---|---|
| **Rohstoffe [INCI Bezeichnungen] (Lieferant)** | **Anteil [%]** |
| Aqua | 68,3 |
| Phenoxyethanol, Methylparaben, Ethylparaben, Butylparaben Propylparaben, Isobutylparaben (Dragocid Liquid, DRAGOCO) | 0,8 |
| Propylene Glycol | 5,0 |

Die Zusammensetzung der Phase B₁ entspricht somit im wesentlichen der Zusammensetzung der Phase B aus den Beispielen 1 und 2; der Anteil von 0,1 % Trisodium Ethylendiamin Tetraacetate wurde jedoch unter entsprechender Erhöhung des Anteils an Wasser (Aqua) ersetzt.

Zur Herstellung der Emulsion werden die Phasen A und B₁ getrennt auf ca. 80 °C erhitzt. Die Phase C wird in der Phase B₁ gelöst. Unter starkem Dispergieren wird die Phase A zu den vereinigten Phasen B₁+C gegeben. Unter ständigem Rühren mit einem Blattrüher wird die Emulsion abgekühlt. Die Phase D wird bei 40 °C zugesetzt. Der pH-Wert der Emulsion wird mit Schwefelsäure auf pH = 5,0 eingestellt.

### Beispiel 4: O/W-Creme mit Citrat-Puffer, aber ohne EDTA (pH 5)

### Rezeptur mit Grünem Tee bei pH 5

Die Creme wird unter Verwendung von vier Phasen A, B₂, C und D gebildet, wobei die Zusammensetzung der Phasen A, C und D der aus den Beispielen 1 und 2 entspricht.

| Zusammensetzung Phase B₂ | |
|---|---|
| **Rohstoffe [INCI Bezeichnungen] (Lieferant)** | **Anteil [%]** |
| Aqua | 67,2 |
| Phenoxyethanol, Methylparaben, Ethylparaben, Butylparaben, Propylparaben, Isobutylparaben (Dragocid Liquid, DRAGOCO) | 0,8 |
| Propylene Glycol | 5,0 |
| Citric Acid | 1,0 |

Die Zusammensetzung der Phase B₂ entspricht somit im wesentlichen der Zusammensetzung der Phase B aus den Beispielen 1 und 2; Anteile von 0,1 % Trisodium Ethylendiamin Tetraacetate und 0,9 % Wasser wurden jedoch durch einen Anteil von 1,0 % Zitronensäure (citric acid) ersetzt.

Zur Herstellung der Emulsion werden die Phasen A und B₂ getrennt auf ca. 80 °C erhitzt. Die Phase C wird in der Phase B₂ gelöst. Unter starkem Dispergieren wird die Phase A zu den vereinigten Phasen B₂+C gegeben. Unter ständigem Rühren mit einem Blattrührer wird die Emulsion abgekühlt. Die Phase D wird bei 40 °C zugesetzt. Der pH-Wert der Emulsion wird mit Natronlauge auf pH = 5,0 eingestellt.

### Beispiel 5: O/W-Creme mit Phospat-Puffer, aber ohne EDTA (pH 5)

### Rezeptur mit Grünem Tee bei pH 5

Die Creme wird unter Verwendung von vier Phasen A, B₃, C und D gebildet, wobei die Zusammensetzung der Phasen A, C und D der aus den Beispielen 1 und 2 entspricht.

| Zusammensetzung Phase B₃ | |
|---|---|
| **Rohstoffe [INCI Bezeichnungen] (Lieferant)** | **Anteil [%]** |
| Aqua | 67,2 |
| Phenoxyethanol, Methylparaben, Ethylparaben, Butylparaben, Propylparaben, Isobutylparaben (Dragocid Liquid, DRAGOCO) | 0,8 |
| Propylene Glycol | 5,0 |
| Sodium Dihydrogenphosphate | 1,0 |

Die Zusammensetzung der Phase B₃ entspricht somit im wesentlichen der Zusammensetzung der Phase B aus den Beispielen 1 und 2; Anteile von 0,1 % Trisodium Ethylendiamin Tetraacetate und 0,9 % Wasser wurden jedoch durch einen Anteil von 1,0 % Natriumhydrogenphosphat (Sodium Dihydrogenphosphate) ersetzt.

Zur Herstellung der Emulsion werden die Phasen A und B₃ getrennt auf ca. 80 °C erhitzt. Die Phase C wird in der Phase B₃ gelöst. Unter starkem Dispergieren wird die Phase A zu den vereinigten Phasen B₃+C gegeben. Unter ständigem Rühren mit einem Blattrührer wird die Emulsion abgekühlt. Die Phase D wird bei 40 °C zugesetzt. Der pH-Wert der Emulsion wird mit Natronlauge auf pH = 5,0 eingestellt.

### Beispiel 6: Lagerungstest der Formulierungen aus den Beispielen 1-5

Es wurden insgesamt 5 Testformulierungen gemäß den obigen Beispielen 1-5 hergestellt. Zur Beobachtung der Alterung dieser Testformulierungen wurden entsprechende Proben im Wärmeschrank bei 40 °C gelagert.

Die Proben wurden in regelmäßigen Zeitabständen von einem Tag visuell begutachtet. Im direkten Vergleich stellte sich die Formulierung aus Beispiel 1 am farbstabilsten dar.

Zusätzlich wurden die Farbveränderungen über die Zeit mit einem Minolta-Chromameter gemessen und als CIE-Lab*-Werte protokolliert. In dem CIELAB-System wird ein dreidimensionales Koordinatenkreuz aus einer a*-Achse (Rot-Grün), einer dazu senkrechten b*-Achse (Blau-Gelb) und einer zu den ab*-Achsen senkrechte L-Achse zur Aussage über die von menschlichen Auge empfundenen Farbunterschiede benutzt. Die Lage des Farbortes auf der L-Achse, d.h. der schon eingangs erwähnte L-Wert, gibt die Helligkeit der Probe an, während der a*-Wert die Lage des Farbortes auf der Rot-Grün-Achse und entsprechend der b*-Wert die Lage auf der Blau-Gelb-Achse angibt. Somit läßt sich jede Farbe durch die Angabe von drei Zahlenwerten klar definieren, und Veränderungen des Farbortes sind ein Maß für die vom menschlichen Auge empfundenen Farbänderungen.

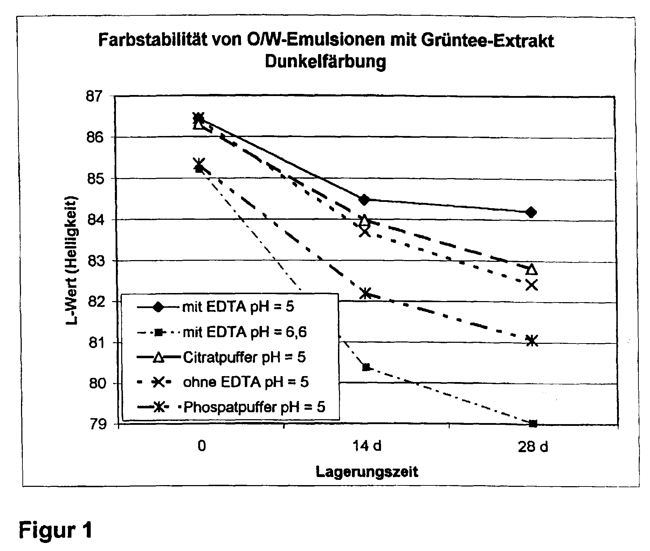

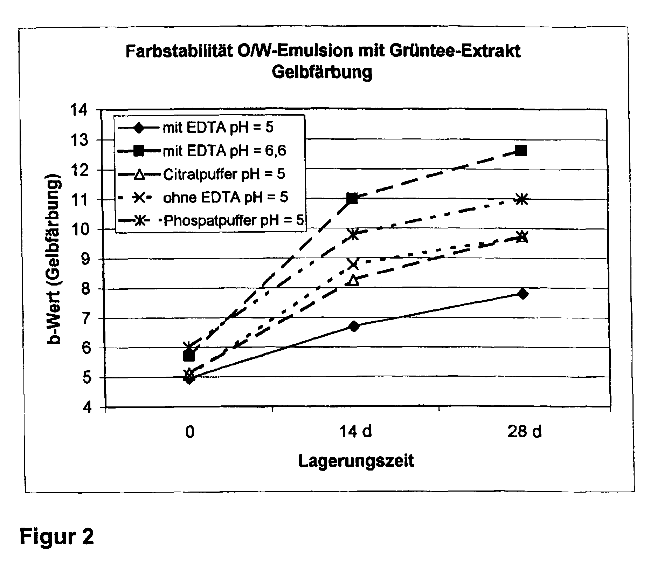

Die Ergebnisse der Messungen der L-Werte und der b*-Werte sind in den Diagrammen gemäß Fig. 1 und Fig. 2 niedergelegt, wobei anzumerken ist, daß ein höherer b*-Wert eine stärkere gelbe Farbe bedeutet und damit ein Maß für die Farbverschiebung in Richtung braun angibt. Die a*-Werte (Rot-Grün) bewegten sich um Null und sind deshalb hier nicht wiedergegeben.

Wie das Diagramm gemäß Figur 1 zeigt, ist die Formulierung aus Beispiel 1 (mit EDTA, pH = 5) am stabilsten, färbt sich also im Vergleich mit den Formulierungen aus den Beispielen 2 - 5 am wenigsten dunkel.

Ohne EDTA zeigen sich auch bei pH = 5 stärkere Dunkelfärbungen. Wird der pH-Wert gemäß Beispiel 2 auf 6,6 erhöht, so verfärbt sich die Formulierung besonders schnell und besonders stark.

Die Puffersysteme Phosphorsäure/Phosphat und Citronensäure/Citrat sind zwar bekannt dafür, daß sie Metalle komplexieren können. Trotzdem wird mit diesen Verbindungen keine zusätzliche Stabilisierung erreicht.

Die anhand des L-Werts dokumentierten Beobachtungen zur Dunkelfärbung entsprechen den Beobachtungen zur Erhöhung der Gelbfärbung, die über den b*-Wert quantifizierbar ist (vgl. das Diagramm gemäß Figur 2).

Die Diagramme gemäß den Figuren 1 und 2 zeigen jeweils, daß die Einstellung eines niedrigen pH-Wertes (z.B. pH 5) kombiniert mit einem Gehalt an einem mehrzähnigen Komplexliganden (z.B. EDTA) ausschlaggebend für die Farbstabilität einer O/W-Formulierung ist.

### Beispiel 7: Vergleichsversuch mit einer nicht-erfin dungsgemäßen O/W-Creme (pH 6,6)

### Rezeptur mit Grünem Tee ohne EDTA bei pH 6,6

Die Beispiele 1-6 zeigten, daß ein zu hoher pH-Wert (pH > 6) die Farbstabilität von Grüntee-enthaltenden Rezepturen ungünstig beeinflußt. Dieses Ergebnis wurde mit der nachfolgend näher erläuterten Creme-Formulierung bestätigt, die ein übliches Emulgatorsystem auf Stearat-Basis umfaßt.

Die Creme wird aus vier Phasen E - H gebildet, deren Zusammensetzung nachstehend angegeben ist:

| Zusammensetzung Phase E | |
|---|---|
| **Rohstoffe [INCI Bezeichnungen] (Lieferant)** | **Anteil [%)** |
| Stearic Acid | 7,0 |
| Cetyl Alcohol | 2,0 |
| Glyceryl Stearate (Dracorin GMS, DRAGOCO) | 1,5 |
| Isopropyl Myristate | 3,0 |
| Stearyl Heptanoate, Stearyl Caprylate (PCL Solid, DRAGOCO) | 2,5 |
| Dimethicone | 0,3 |

| Zusammensetzung Phase F | |
|---|---|
| Aqua | 74,7 |
| Sodium Dihydroxycetyl Phosphate (Dragophos S, DRAGOCO) | 2,5 |
| Propylene Glycol | 5,0 |
| Methylparaben, Sorbic Acid, Dehydroacetic Acid, Propylparaben (Neo-Dragocid Pulver, DRAGOCO) | 0,3 |
| Sodium Hydroxide | 0,7 |

| Zusammensetzung Phase G | |
|---|---|
| Aqua | 1,0 |
| Camellia Sinensis (Grüner Tee Extra, DRAGOCO) | 0,1 |

| Zusammensetzung Phase H | |
|---|---|
| Fragrance | 0,4 |

Zur Herstellung der Emulsion werden die Phasen E und F getrennt auf ca. 80 °C erhitzt. Die Phase G wird in der Phase F gelöst. Unter starkem Dispergieren wird die Phase E zu den vereinigten Phasen F+G gegeben. Unter ständigem Rühren mit einem Blattrührer wird die Emulsion abgekühlt. Die Phase H wird bei 40 °C zugesetzt. Der pH-Wert der Emulsion beträgt 6,6.

Diese Emulsion ist bereits nach Ihrer Herstellung leicht braun. Bei der Lagerung kommt es zu einer beträchtlichen Nachdunkelung.

## Patentansprüche

1. O/W-Emulsion mit einem Gehalt an Grüntee-Extrakt insbesondere zur kosmetischen oder pharmazeutischen Verwendung, dadurch gekennzeichnet,
- daß der pH-Wert der Emulsion zwischen 2 und 6 liegt,
- daß die Emulsion einen mehrzähnigen Komplexliganden umfaßt und
- daß die Emulsion einen L-Wert über 81 aufweist.

2. Emulsion nach Anspruch 1, dadurch gekennzeichnet, daß der pH-Wert der Formulierung zwischen 4,5 und 5,5 liegt.

3. Emulsion nach Anspruch 1 oder 2, dadurch gekennzeichnet, daß der Komplexligand Ethylendiamintetraessigsäure (EDTA) oder Ethylendiamintetraacetat ist.

4. Emulsion nach einem der vorangehenden Ansprüche, dadurch gekennzeichnet, daß die Emulsion hautpflegende Substanzen umfaßt.

5. Verwendung eines mehrzähnigen Komplexliganden als Mittel zur Verzögerung oder Verhinderung der Verfärbung einer Wasser und einen Grüntee-Extrakt umfassenden, auf einen pH-Wert zwischen 2 und 6 eingestellten O/W-Emulsion.

6. Verfahren zur farblichen Stabilisierung einer Wasser und einen Grüntee-Extrakt umfassenden O/W-Emulsion, dadurch gekennzeichnet, daß
- der pH der Emulsion auf einen Wert zwischen 2 und 6 eingestellt wird und
- die Emulsion mit einem mehrzähnigen Komplexliganden versetzt wird.
